# FASCICULE DE BREVET EUROPEEN

(11) **EP 4 479 132 B1**
(45) Date de publication et mention de la délivrance du brevet: **07.10.2026**
(21) Numéro de dépôt: 23704190.0
(22) Date de dépôt: 15.02.2023
(51) Int. Cl.: A61N 5/06, A61M 21/02

(54) **DISPOSITIF POUR LE TRAITEMENT D'UN SUJET**
VORRICHTUNG ZUR BEHANDLUNG EINER PERSON
DEVICE FOR TREATING A SUBJECT

(30) Priorité: 15.02.2022 FR 2201307
(43) Date de publication de la demande: 25.12.2024
(73) Titulaire: Delgado, Yannick, 44360 Vigneux de Bretagne (FR)
(72) Inventeur: Delgado, Yannick, 44360 Vigneux de Bretagne (FR)
(74) Mandataire: Jacobacci & Partners France
(86) Numéro de dépôt international: PCT/EP2023/053702
(87) Numéro de publication internationale: WO 2023/156424

(56) Documents cités:
- EP-A1- 2 735 337
- JP-A- 2008 272 273
- US-B1- 7 537 576

## Description

### Domaine technique de l'invention

La présente invention concerne le domaine technique des dispositifs pour le traitement d'un sujet (humain ou animal) par le biais d'une combinaison de différentes techniques, à savoir notamment la lithothérapie, la chromothérapie, la luminothérapie ou la musicothérapie.

### Etat de la technique

La médecine non-conventionnelle, dite encore « médecine douce », englobe une grande variété de méthodes de traitement.

On connait par exemple des méthodes de traitement qui utilisent la chromothérapie (utilisation des couleurs), la lithothérapie (utilisation de cristaux, roches et minéraux), la musicothérapie (utilisation de la musique) et l'aromathérapie (utilisation d'huiles essentielles de plantes). Par exemple , JP 2008 272273 divulgue un dispositif de traitement qui comprend une enveloppe en forme de pyramidion, dans lequel sont rapportés un cristal et une source de lumière froide.

Le praticien utilise souvent un dispositif dédié pour la mise en œuvre de ces techniques sur le sujet à traiter.

Mais la plupart des dispositifs actuels sont adaptés à la mise en oeuvre d'un seul type de traitement.

Il existe par conséquent un besoin de nouveaux dispositifs pour le traitement d'un sujet, qui seraient aptes à combiner différentes techniques de médecines non-conventionnelles.

### Présentation de l'invention

Afin de remédier à l'inconvénient précité de l'état de la technique, la présente invention propose un dispositif pour le traitement d'un sujet, comprenant une enveloppe, en forme de pyramidion, qui comporte un apex, une base à l'opposé de l'apex et un axe central passant par ledit apex.

L'enveloppe délimite un volume interne dans lequel sont rapportés des moyens lumineux comportant un corps délimitant une chambre directionnelle qui est agencée coaxialement audit axe central et qui débouche du côté dudit apex.

La chambre directionnelle comporte :
- un cristal de quartz, orienté coaxialement audit axe central, et
- une source de lumière froide, de préférence de couleur blanche, à l'opposé de l'apex, pour la production d'un faisceau de lumière froide destiné à traverser ledit cristal de quartz, de sorte à obtenir un faisceau lumineux de traitement qui est régis par la traversée dudit cristal de quartz et qui est émis au niveau dudit apex.

Le dispositif de traitement comprend des moyens de commande, pour piloter le fonctionnement desdits moyens lumineux.

Un tel dispositif de traitement est ainsi adapté à la mise en oeuvre d'une combinaison de différentes techniques reconnues en médecine non-conventionnelle, à savoir notamment la lithothérapie, la chromothérapie, la luminothérapie ou la musicothérapie.

D'autres caractéristiques non limitatives et avantageuses du produit conforme à l'invention, prises individuellement ou selon toutes les combinaisons techniquement possibles, sont les suivantes :
- l'enveloppe présente un ratio cote de côté sur cote en hauteur allant de 1,5 à 1,8, avec par exemple une cote de côté allant de 225 à 245 mm, et une cote en hauteur allant de 140 à 180 mm ;
- la chambre directionnelle intègre un filtre optique qui est implanté entre le cristal de quartz et la source de lumière froide ;
- les moyens de commande comportent des moyens de raccordement à un dispositif de traitement maître comprenant : a) des moyens lumineux comprenant plusieurs unités lumineuses qui sont chacune destinées à émettre un faisceau lumineux de traitement ayant un spectre lumineux prédéfini, lesquelles unités lumineuses comportent chacune un corps qui délimite une chambre directionnelle dans laquelle sont rapportés un cristal de quartz et des moyens pour la production d'un faisceau de lumière froide destiné à traverser ledit cristal de quartz, lequel corps comporte encore une ouverture au niveau de laquelle est émis ledit faisceau lumineux de traitement, provenant desdits moyens de production et régis par la traversée dudit cristal de quartz, lesdites unités lumineuses étant chacune adaptées à être disposées de sorte à orienter leurs ouvertures respectives en regard d'un point du corps dudit sujet, b) des moyens sonores, destinés à diffuser des sons, et c) des moyens de commande, pour piloter et pour coordonner le fonctionnement desdits moyens lumineux et desdits moyens sonores ;
- ledit dispositif de traitement comporte une plaque qui est munie de premiers symboles et qui est traversé par le cristal de quartz, lequel dispositif de traitement comporte des moyens de raccordement mécaniques à une table de soin comportant des seconds symboles, identiques auxdits premiers symboles ;
- ledit dispositif de traitement comporte des moyens de raccordement électronique à une interface homme-machine, par exemple un dispositif électronique portatif, pour le pilotage desdits moyens de commande ;
- l'enveloppe comporte une pièce d'apex amovible, orienté coaxialement audit axe central, de sorte à obtenir une ouverture dans ladite enveloppe au niveau de laquelle débouche la chambre directionnelle et ledit cristal de quartz et au niveau de laquelle est émis le faisceau lumineux de traitement, laquelle enveloppe comporte avantageusement une réservation dans sa base, pour loger ladite pièce d'apex amovible ;
- le cristal de quartz consiste en un cristal de quartz multifacettes.

La présente invention concerne également un système de traitement d'un sujet, comprenant :
- un dispositif de traitement selon l'invention, et
- un dispositif de traitement maître comprenant :
   a) des moyens lumineux comprenant plusieurs unités lumineuses qui sont chacune destinées à émettre un faisceau lumineux de traitement ayant un spectre lumineux prédéfini,
      lesquelles unités lumineuses comportent chacune un corps qui délimite une chambre directionnelle dans laquelle sont rapportés :
         - un cristal de quartz et
         - des moyens pour la production d'un faisceau de lumière froide destiné à traverser ledit cristal de quartz,
      lequel corps comporte encore une ouverture au niveau de laquelle est émis ledit faisceau lumineux de traitement, provenant desdits moyens de production et régis par la traversée dudit cristal de quartz,
      lesdites unités lumineuses étant chacune adaptées à être disposées de sorte à orienter leurs ouvertures respectives en regard d'un point du corps dudit sujet,
   b) des moyens sonores, destinés à diffuser des sons, et
   c) des moyens de commande, pour piloter et pour coordonner le fonctionnement desdits moyens lumineux et desdits moyens sonores.

Le système de traitement selon l'invention comprend encore avantageusement la table de soin précitée.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue générale et en perspective d'un dispositif conforme à l'invention, pour le traitement d'un sujet ;
[Fig. 2] est une vue générale et en perspective du dispositif de traitement selon la figure 1, et dans laquelle une pièce d'apex amovible est retirée ;
[Fig. 3] est une vue générale et schématique, selon un plan de coupe vertical, du dispositif de traitement selon la figure 1 ;
[Fig. 4] est une vue générale et en perspective du dispositif de traitement selon la figure 1, vu par dessous ;
[Fig. 5] est une vue générale et en perspective d'un dispositif de traitement maître adapté à être raccordé au dispositif de traitement selon l'invention.

Il est à noter que, sur ces figures, les éléments structurels et/ou fonctionnels communs aux différentes variantes peuvent présenter les mêmes références.

Tel que représenté sur les figures, la présente invention concerne ainsi un dispositif 1 pour le traitement d'un sujet, dit encore « dispositif de traitement 1 ».

Le dispositif de traitement 1 est destiné à être utilisé par un praticien pour le traitement d'un sujet.

Par « traitement », on entend un traitement médical conventionnel et/ou un traitement médical non-conventionnel (dit encore de « médecine douce »).

Par « sujet », on entend avantageusement un être vertébré, et de préférence encore un être humain ou animal.

Ce dispositif de traitement 1 selon l'invention est par exemple destiné :
- aux acuponcteurs, ostéopathes, kinésithérapeutes, énergéticiens, naturopathes, sophrologues, réflexologues et autres ;
- au corps médical dans sa globalité ;
- aux professionnels de soins palliatifs ;
- aux centres hospitaliers ;
- aux laboratoires de recherche médicale et bio médicale ;
- aux centres de soins naturels et de remise en forme ;
- aux thalassothérapies ;
- aux centres de massage.

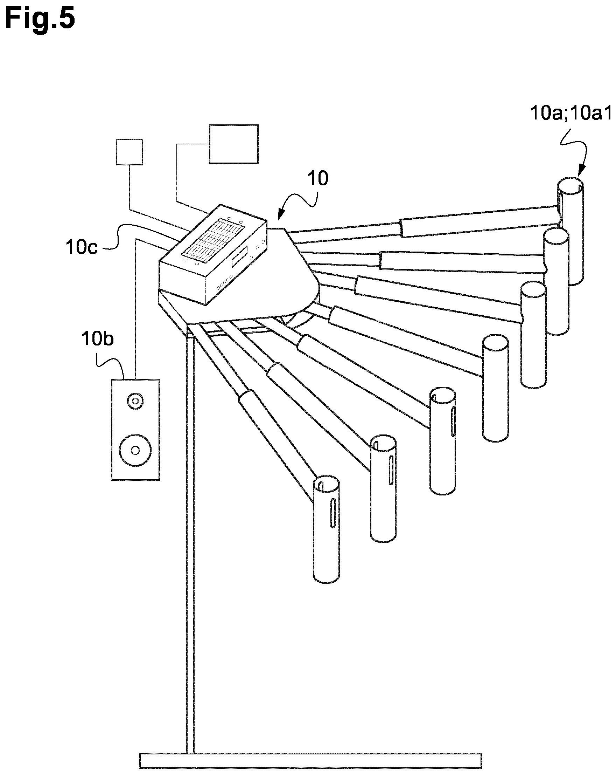

En l'espèce, le dispositif de traitement 1 selon l'invention est avantageusement adapté à la mise en oeuvre d'un ensemble de traitements médicaux non-conventionnels et non invasifs, d'une manière pilotée et coordonnée, à savoir choisis par exemple parmi :
- la luminothérapie, par l'utilisation de flux lumineux,
- la chromothérapie, par l'utilisation de couleurs,
- la lithothérapie, par l'utilisation de cristaux de quartz,
- voire la musicothérapie, par l'utilisation de thèmes musicaux choisis qui sont avantageusement embarqués et à fréquence définie, générés avantageusement par un dispositif de traitement maître décrit par la suite en relation avec la figure 5.

Pour cela, le dispositif de traitement 1, selon l'invention, comprend une enveloppe 2 qui délimite un volume interne 3 dans lequel sont rapportés des moyens lumineux 4 coopérant avec des moyens de commande 5 (adaptés à piloter le fonctionnement de ces moyens lumineux 4).

### Enveloppe

L'enveloppe 2 est en forme de pyramidion.

Par « pyramidion », on entend avantageusement une petite pyramide quadrangulaire qui couronne habituellement le sommet d'un monument (par exemple une pyramide ou un obélisque).

L'enveloppe 2 consiste ainsi avantageusement en une pyramide quadrangulaire à cinq facettes, à savoir :
- une base 21, en forme de quadrilatère (avantageusement carrée), et
- quatre facettes latérales triangulaire 22.

L'enveloppe 2 comporte encore un apex 23 qui est opposé à la base 21 et qui forme avantageusement le sommet de l'enveloppe 2.

L'enveloppe 2 comporte également un axe central 2' passant par l'apex 23 précité.

De manière générale, le pyramidion présente différentes cotes, notamment :
- une cote de côté, correspondant à la cote des arêtes de la base 21, et
- une cote en hauteur, correspondant à la cote allant de la base 21 jusqu'à l'apex 23. Le ratio cote de côté sur cote en hauteur est avantageusement de 1,5 à 1,8.

Par exemple encore, l'enveloppe 2 présente les cotes suivantes :
- une cote de côté allant de 225 à 245 mm, et
- une cote en hauteur allant de 140 à 180 mm.

De préférence encore, l'enveloppe 2 comporte une pièce d'apex amovible 2a, orientée coaxialement audit axe central 2'.

La pièce d'apex amovible 2a présente également avantageusement une forme pyramidale quadrangulaire.

L'enveloppe 2 comporte alors avantageusement :
- la pièce d'apex amovible 2a, portant l'apex 23 et formée par une partie supérieure des quatre facettes latérales triangulaires 22, et
- une embase 2b, en forme de tronc de pyramide, formée par la base 21 et une partie inférieure des quatre facettes latérales triangulaires 22.

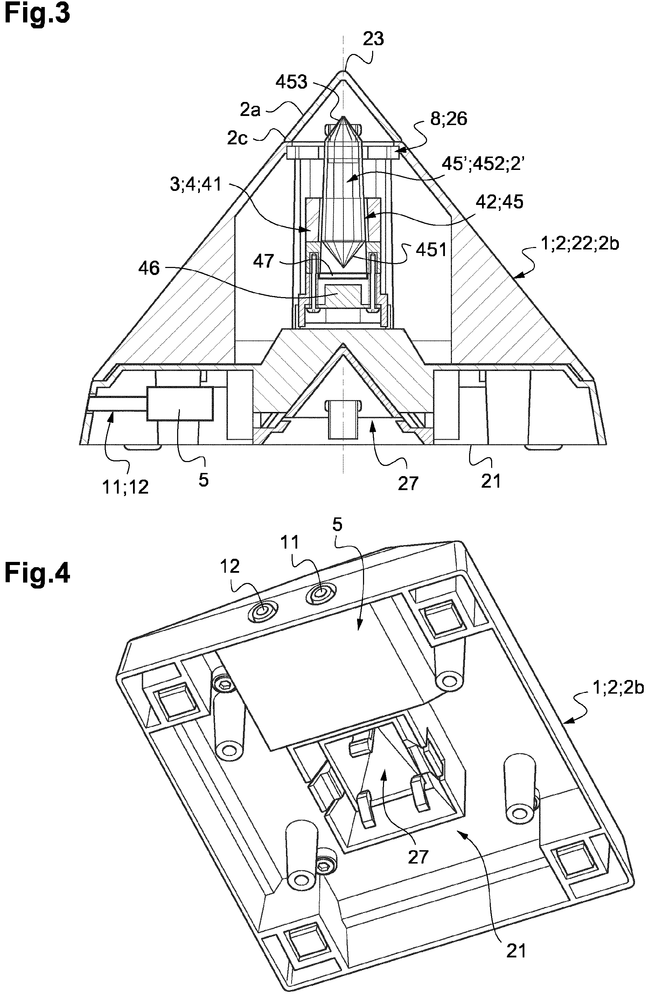

De préférence, comme illustré sur la figure 3, la pièce d'apex amovible 2a et l'embase 2b coopèrent par des moyens d'assemblage amovible 2c, par exemple par emboîtement par déformation élastique (dit encore par clipsage).

L'embase 2b enveloppe avantageusement les moyens lumineux 4 et les moyens de commande 5.

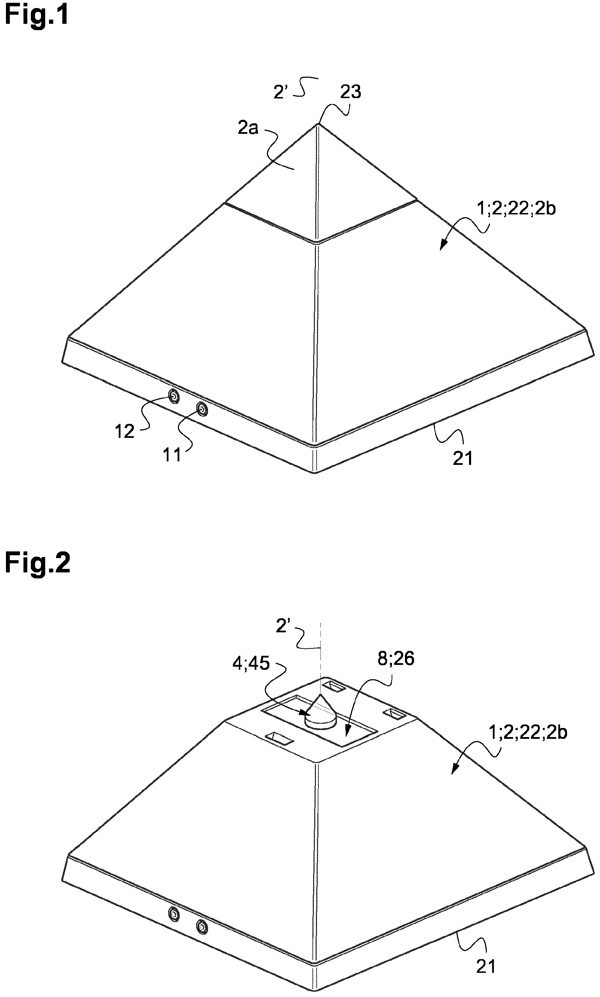

Tel que représenté sur la figure 2, la pièce d'apex amovible 2a permet d'obtenir une ouverture 26 dans cette enveloppe 2 (et en particulier au niveau de l'embase 2b) au niveau de laquelle débouche les moyens lumineux 4.

La pièce d'apex amovible 2a forme ainsi un couvercle qui est adapté à obturer l'ouverture 26 de cette enveloppe 2.

Dans ce cas, l'enveloppe 2 (et en particulier son embase 2b) comporte avantageusement une réservation 27 dans sa base 21, pour loger la pièce d'apex amovible 2a lorsque l'ouverture 26 est libérée (voir les figures 3 et 4).

### Moyens lumineux

Les moyens lumineux 4 comporte un corps 41 délimitant une chambre directionnelle 42 qui est agencée coaxialement à l'axe central 2' et qui débouche du côté de l'apex 23.

De préférence, le corps 41 est solidarisé avec l'embase 2b, au niveau de son ouverture 26.

Le corps 41 présente avantageusement une forme générale tubulaire qui délimite la chambre directionnelle 42.

En l'espèce, la chambre directionnelle 42 débouche au niveau de l'ouverture 26 portée par l'embase 2b de l'enveloppe 2.

La chambre directionnelle 42 comporte :
- un cristal de quartz 45, orienté coaxialement à l'axe central 2', et
- une source de lumière froide 46, à l'opposé de l'apex 23, pour la production d'un faisceau de lumière froide destiné à traverser le cristal de quartz 45.

Le cristal de quartz 45 consiste avantageusement en un cristal de quartz 45 multifacettes (par exemple à douze facettes).

De manière générale, par « quartz », on entend l'espèce minérale du groupe des silicates, sous-groupe des tectosilicates, composée de dioxyde de silicium (silice), avec des traces de différents éléments.

Le cristal de quartz 45 est avantageusement incolore. Ce cristal de quartz 45 est avantageusement technologiquement clair ou optiquement clair.

Ce cristal de quartz 45 peut être choisi et sélectionné par exemple parmi les cristaux de roche translucides, sans inclusion ou à faible inclusion.

Ce cristal de quartz 45 présente avantageusement une forme taillée pour former trois parties coaxiales polyédriques, selon un axe longitudinal 45', à savoir :
- une première partie terminale 451, conique et large, pour former une pointe femelle, ici du côté de la source de lumière froide 46 et avantageusement logée au sein de l'embase 2b,
- une partie intermédiaire 452, tronconique, et
- une seconde partie terminale 453, conique et étroite, pour former une pointe mâle, ici du côté de l'apex 23 et avantageusement situé à l'extérieur de l'embase 2b (avantageusement sous la pièce d'apex amovible 2a).

La partie intermédiaire 452 tronconique comporte avantageusement une section décroissante depuis la première partie terminale 451 jusqu'à la seconde partie terminale 453.

Les deux parties terminales 451, 453 comportent avantageusement un angle compris entre 40° et 55°.

De manière générale, le cristal de quartz 45 est ici agencé de sorte que son axe longitudinal 45' soit coaxial à l'axe central 2' de l'enveloppe 2.

Le cristal de quartz 45 traverse ici l'ouverture 26 portée par l'embase 2b de l'enveloppe 2, de sorte ce que ce cristal de quartz 45 s'étend de part et d'autre de cette ouverture 26.

Un tronçon de la partie intermédiaire 452 et la seconde partie terminale 453 sont alors libres, en saillie de l'ouverture 26.

La source de lumière froide 46 génère de préférence une lumière froide de couleur blanche.

Le faisceau de lumière froide est destiné à traverser le cristal de quartz 45, de sorte à obtenir un faisceau lumineux de traitement qui est régis par la traversée de ce cristal de quartz 45 et qui est émis au niveau de l'apex 23.

La source de lumière froide 46 est avantageusement choisie parmi les lampes émettant une lumière froide de couleur blanche, ayant éventuellement un rendement ultraviolet dans le domaine du visible et dépourvue d'infrarouge.

La source de lumière froide 46 a avantageusement un faible rendement lumineux, par exemple inférieur à 90 lumens.

Par « lumière froide », on entend une lumière émise par luminescence et avantageusement par électroluminescence, par exemple par le biais d'une diode électroluminescente (LED).

Par « couleur blanche », on entend une lumière dont le spectre couvre uniformément, ou au moins approximativement uniformément, le spectre visible.

Par « spectre visible » ou « spectre optique », on entend la partie du spectre électromagnétique visible pour l'œil humain, c'est-à-dire une représentation de l'ensemble des composantes monochromatiques de la lumière visible.

Ce spectre visible est en particulier délimité par les longueurs d'onde allant de 380 nm à 780 nm, dans le vide.

Par « dépourvue d'infrarouge », on entend en particulier une lumière dépourvue de rayons électromagnétiques dont la longueur d'onde est supérieure à celle de la lumière visible.

Par « rendement ultraviolet », on entend la présence éventuelle de rayonnement ayant une longueur d'onde inférieure à 380 nm, généralement comprise entre 10 nm et 380 nm.

De manière générale, la chambre directionnelle 42 intègre avantageusement un filtre optique 47 qui est implanté entre le cristal de quartz 45 et la source de lumière froide 46.

Le filtre optique 47 est avantageusement destiné à définir la couleur du faisceau lumineux de traitement (avantageusement par un phénomène de filtrage par absorption).

### Moyens de commande

Les moyens de commande 5 consistent par exemple en un circuit imprimé sur lequel se trouve un microcontrôleur associé à différentes interfaces entrée/sortie.

Le microcontrôleur peut être programmé pour analyser et produire des signaux électriques, de manière à effectuer des tâches très diverses, à partir d'un programme d'ordinateur adapté à façon.

Les moyens de commande 5 comportent avantageusement des premiers moyens de raccordement 11, adaptés à un raccordement à un dispositif de traitement maître 10.

Un dispositif de traitement maître 10 est par exemple illustré sur la figure 5 et décrit en détails dans le document FR-3 045 392.

De manière générale, le dispositif de traitement maître 10 comprend avantageusement :
a) des moyens lumineux 10a comprenant plusieurs unités lumineuses 10a1 qui sont chacune destinées à émettre un faisceau lumineux de traitement ayant un spectre lumineux prédéfini,
b) des moyens sonores 10b, destinés à diffuser des sons, et
c) des moyens de commande 10c, pour piloter et pour coordonner le fonctionnement des moyens lumineux 10a et des moyens sonores 10b.

Les unités lumineuses 10a1 comportent chacune un corps qui délimite une chambre directionnelle dans laquelle sont rapportés :
- un cristal de quartz, et
- des moyens pour la production d'un faisceau de lumière froide destiné à traverser ledit cristal de quartz.

Le corps comporte encore une ouverture au niveau de laquelle est émis ledit faisceau lumineux de traitement, provenant desdits moyens de production et régis par la traversée dudit cristal de quartz.

Les unités lumineuses 10a1 sont chacune adaptées à être disposées de sorte à orienter leurs ouvertures respectives en regard d'un point du corps du sujet.

Dans ce cas, le dispositif de traitement 1 est avantageusement situé sous la table de soin sur laquelle repose le sujet.

Le dispositif de traitement 1 peut être orienté également sur différents points en inverse, ou en opposition, du corps du sujet.

Le dispositif de traitement 1 comporte encore avantageusement des moyens de raccordement électronique 12 adaptés à un raccordement à une interface homme-machine (non représentée), par exemple un dispositif électronique portatif, pour le pilotage des moyens de commande 5.

### Plaque

De préférence, le dispositif de traitement 1 comporte une plaque 8 qui est munie de premiers symboles et qui est traversé par le cristal de quartz 45.

Le dispositif de traitement 1 comporte des moyens de raccordement mécaniques à une table de soin (non représentée) comportant des seconds symboles, identiques auxdits premiers symboles.

Par « moyens de raccordement mécaniques », on entend avantageusement une pièce allongée, par exemple un câble métallique, qui est raccordé à la plaque 8 et à la table de soin.

### Système de traitement d'un sujet

La présente invention concerne encore avantageusement un système de traitement d'un sujet, comprenant :
- le dispositif de traitement 1 selon l'invention, formant un dispositif de traitement auxiliaire 1,
- le dispositif de traitement maître 10 précité, et
- éventuellement la table de soin précitée.

### Mise en œuvre

Le dispositif de traitement 1 fonctionne avantageusement en simultané avec le dispositif de traitement maître 10, ou indépendamment du dispositif de traitement maître 10.

Il est actionné en fonction d'une programmation définie qui, sans être limité par une quelque théorie, permet :
- une information vibratoire par champ de torsion traversant la table de soin précitée pour une action sur le corps sujet, ou
- une information vibratoire par champ mécanique et de torsion sur la table de soin précitée pour une action sur le corps sujet.

Bien entendu, diverses autres modifications peuvent être apportées à l'invention dans le cadre des revendications annexées.

## Revendications

1. Système de traitement d'un sujet, comprenant :
- un dispositif de traitement maître (10) comprenant :
a) des moyens lumineux (10a) comprenant plusieurs unités lumineuses (10a1) qui sont chacune destinées à émettre un faisceau lumineux de traitement ayant un spectre lumineux prédéfini, lesquelles unités lumineuses (10a1) comportent chacune un corps qui délimite une chambre directionnelle dans laquelle sont rapportés :
- un premier cristal de quartz et
- des premiers moyens pour la production d'un faisceau de lumière froide destiné à traverser ledit cristal de quartz, lequel corps comporte encore une ouverture au niveau de laquelle est émis ledit faisceau lumineux de traitement, provenant desdits moyens de production et régis par la traversée dudit premier cristal de quartz, lesdites unités lumineuses (10a1) étant chacune adaptées à être disposées de sorte à orienter leurs ouvertures respectives en regard d'un point du corps dudit sujet,
b) des moyens sonores (10b), destinés à diffuser des sons, et
c) des moyens de commande (10c), pour piloter et pour coordonner le fonctionnement desdits moyens lumineux (10a) et desdits moyens sonores (10b),
et
- un dispositif de traitement (1), formant un dispositif de traitement auxiliaire (1), ledit dispositif de traitement (1) comprenant une enveloppe (2), en forme de pyramidion, qui comporte un apex (23), une base (21) à l'opposé de l'apex (23) et un axe central (2') passant par ledit apex (23),
laquelle enveloppe (2) délimite un volume interne (3) dans lequel sont rapportés des moyens lumineux (4) comportant un corps (41) délimitant une chambre directionnelle (42) qui est agencée coaxialement audit axe central (2') et qui débouche du côté dudit apex (23),
laquelle chambre directionnelle (42) comporte :
- un deuxième cristal de quartz (45), orienté coaxialement audit axe central (2'), et
- une deuxième source de lumière froide (46), de préférence de couleur blanche, à l'opposé de l'apex (23), pour la production d'un faisceau de lumière froide destiné à traverser ledit deuxième cristal de quartz (45),
de sorte à obtenir un faisceau lumineux de traitement qui est régis par la traversée dudit deuxième cristal de quartz (45) et qui est émis au niveau dudit apex (23),
lequel dispositif de traitement (1) comprend des moyens de commande (5), pour piloter le fonctionnement desdits moyens lumineux (4),
lesquels moyens de commande (5) comportent des moyens de raccordement (11) audit dispositif de traitement maître (10).

2. Système de traitement d'un sujet, selon la revendication 1, où l'enveloppe (2) présente un ratio cote de côté sur cote en hauteur allant de 1,5 à 1,8.

3. Système de traitement d'un sujet, selon la revendication 2, où l'enveloppe (2) présente :
- une cote de côté allant de 225 à 245 mm, et
- une cote en hauteur allant de 140 à 180 mm.

4. Système de traitement d'un sujet, selon l'une quelconque des revendications 1 à 3, où la chambre directionnelle (42) intègre un filtre optique (47) qui est implanté entre le deuxième cristal de quartz (45) et la deuxième source de lumière froide (46).

5. Système de traitement d'un sujet, selon l'une quelconque des revendications 1 à 4, où ledit dispositif de traitement (1) comporte une plaque (8) qui est munie de premiers symboles et qui est traversé par le deuxième cristal de quartz (45), lequel dispositif de traitement (1) comporte des moyens de raccordement mécaniques à une table de soin comportant des seconds symboles, identiques auxdits premiers symboles.

6. Système de traitement d'un sujet, selon l'une quelconque des revendications 1 à 5, où ledit dispositif de traitement (1) comporte des moyens de raccordement électronique (12) à une interface homme-machine, par exemple un dispositif électronique portatif, pour le pilotage desdits moyens de commande (5).

7. Système de traitement d'un sujet, selon l'une quelconque des revendications 1 à 6, où l'enveloppe (2) comporte une pièce d'apex amovible (2a), orienté coaxialement audit axe central (2'), de sorte à obtenir une ouverture dans ladite enveloppe (2) au niveau de laquelle débouche la chambre directionnelle (42) et ledit deuxième cristal de quartz (45) et au niveau de laquelle est émis le faisceau lumineux de traitement.

8. Système de traitement d'un sujet, selon la revendication 7, où l'enveloppe (2) comporte une réservation (27) dans sa base (21), pour loger ladite pièce d'apex amovible (2a).

9. Système de traitement d'un sujet, selon l'une quelconque des revendications 1 à 8, où le deuxième cristal de quartz (45) consiste en un cristal de quartz (45) multifacettes.

10. Système de traitement d'un sujet, selon la revendication 9 en combinaison avec la revendication 5, comprenant également ladite table de soin.

## Patentansprüche

1. System zur Behandlung einer Person mit
- einer Hauptbehandlungsvorrichtung (10) mit
a) Leuchtmitteln (10a) mit mehreren Leuchteinheiten (10a1), die jeweils dazu bestimmt sind, einen Behandlungslichtstrahl mit einem vorbestimmten Lichtspektrum auszusenden,
wobei die Leuchteinheiten (10a1) jeweils einen Körper aufweisen, der eine Richtkammer begrenzt, in der:
- ein erster Quarzkristall und
- erste Mittel zum Erzeugen eines Strahls kalten Lichts, der den Quarzkristall durchqueren soll,
angeordnet sind,
wobei der Körper außerdem eine Öffnung aufweist, auf deren Höhe der von den Erzeugungsmitteln kommende und durch das Durchqueren des ersten Quarzkristalls geregelte Behandlungslichtstrahl ausgesandt wird,
wobei die Leuchteinheiten (10a1) jeweils dazu ausgelegt sind, so angeordnet zu werden, daß deren jeweilige Öffnungen auf einen Punkt des Körpers der Person gerichtet sind,
b) akustischen Mitteln (10b), die dazu bestimmt sind, Töne auszusenden, und
c) Steuerungsmitteln (10c) zum Steuern und zum Koordinieren des Betriebs der Leuchtmittel (10a) und der akustischen Mittel (10b),
und
- einer Behandlungsvorrichtung (1), die eine Hilfsbehandlungsvorrichtung (1) bildet,
wobei die Behandlungsvorrichtung (1) eine Hülle (2) in Form einer Pyramide, die eine Spitze (23), eine zur Spitze (23) entgegengesetzte Basis (21) und eine durch die Spitze (23) gehende Mittelachse (2') hat, aufweist,
wobei die Hülle (2) ein inneres Volumen (3) begrenzt, in dem Leuchtmittel (4) mit einem Körper (41) angeordnet sind, der eine Richtkammer (42) begrenzt, die koaxial zur Mittelachse (2') eingerichtet ist und zur Spitze (23) hin offen ist,
wobei die Richtkammer (42)
- einen zur Mittelachse (2') koaxial ausgerichteten zweiten Quarzkristall (45) und
- eine zur Spitze (23) entgegengesetzte zweite Quelle (46) kalten Lichts, vorzugsweise weißer Farbe, für das Erzeugen eines Strahls kalten Lichts, der dazu bestimmt ist, den zweiten Quarzkristall (45) zu durchqueren,
aufweist,
um einen Behandlungslichtstrahl zu erhalten, der durch das Durchqueren des zweiten Quarzkristalls (45) geregelt wird und der in Höhe der Spitze (23) ausgestrahlt wird,
wobei die Behandlungsvorrichtung (1) Steuerungsmittel (5) aufweist, um den Betrieb der Leuchtmittel (4) zu steuern,
wobei die Steuerungsmittel (5) Mittel (11) für einen Anschluß an die Hauptbehandlungsvorrichtung (10) aufweisen.

2. System zur Behandlung einer Person gemäß Anspruch 1, bei dem die Hülle (2) ein Verhältnis des Seitenmaßes zum Höhenmaß von 1,5 bis 1,8 aufweist.

3. System zur Behandlung einer Person gemäß Anspruch 2, bei dem die Hülle (2)
- ein Seitenmaß von 225 bis 245 mm und
- ein Höhenmaß von 140 bis 180 mm
aufweist.

4. System zur Behandlung einer Person gemäß einem der Ansprüche 1 bis 3, bei dem die Richtkammer (42) einen optischen Filter (47) beinhaltet, der zwischen dem zweiten Quarzkristall (45) und der zweiten Quelle (46) kalten Lichts eingesetzt ist.

5. System zur Behandlung einer Person gemäß einem der Ansprüche 1 bis 4, bei dem die Behandlungsvorrichtung (1) eine Platte (8) aufweist, die mit ersten Symbolen versehen ist und die vom zweiten Quarzkristall (45) durchdrungen wird, wobei die Behandlungsvorrichtung (1) mechanische Mittel für einen Anschluß an einen Behandlungstisch aufweist, der mit den ersten Symbolen identische zweite Symbole aufweist.

6. System zur Behandlung einer Person gemäß einem der Ansprüche 1 bis 5, bei dem die Behandlungsvorrichtung (1) Mittel (12) für einen elektronischen Anschluß an eine Mensch-Maschine-Schnittstelle, zum Beispiel eine tragbare elektronische Vorrichtung zum Steuern der Steuerungsmittel (5), aufweist.

7. System zur Behandlung einer Person gemäß einem der Ansprüche 1 bis 6, bei dem die Hülle (2) ein abnehmbares Spitzenstück (2a) aufweist, das zur Mittelachse (2') koaxial ausgerichtet ist, um in der Hülle (2) eine Öffnung zu erhalten, auf deren Höhe die Richtkammer (42) und der zweite Quarzkristall (45) münden und auf deren Höhe der Behandlungslichtstrahl ausgesandt wird.

8. System zur Behandlung einer Person gemäß Anspruch 7, bei dem die Hülle (2) in ihrer Basis (21) eine Ausnehmung (27) aufweist, um das abnehmbare Spitzenstück (2a) unterzubringen.

9. System zur Behandlung einer Person gemäß einem der Ansprüche 1 bis 8, bei dem der zweite Quarzkristall (45) aus einem Quarzkristall (45) mit mehreren Flächen besteht.

10. System zur Behandlung einer Person gemäß Anspruch 9 in Verbindung mit Anspruch 5, das auch den Behandlungstisch aufweist.

## Claims

1. A system for treating a subject, comprising:
- a master treatment device (10) comprising:
a) light means (10a) comprising several light units (10a1) which are each intended to emit a treatment light beam having a predefined light spectrum,
which light units (10a1) each include a body that delimits a directional chamber in which are attached:
- a first quartz crystal and
- first means for producing a cold light beam intended to pass through said quartz crystal,
which body further includes an opening at which said treatment light beam is emitted, coming from said production means and governed by the passage through said First quartz crystal,
said light units (10a1) being each adapted to be arranged so as to orient their respective openings facing a point of the body of said subject,
b) sound means (10b), intended to diffuse sounds, and
c) control means (10c), for driving and coordinating the operation of said light means (10a) and said sound means (10b),
and
- a treatment device (1), forming an auxiliary treatment device (1), said treatment device (1) comprising a pyramidion-shaped envelope (2), which includes an apex (23), a base (21) opposite the apex (23) and a central axis (2') passing through said apex (23),
wherein the envelope (2) delimits an inner volume (3) in which light means (4) are attached, including a body (41) delimiting a directional chamber (42) which is arranged coaxially to said central axis (2') and which opens on the side of said apex (23),
wherein the directional chamber (42) includes:
- a second quartz crystal (45), oriented coaxially to said central axis (2'), and
- a second cold light source (46), preferably of white colour, opposite the apex (23),
for the production of a beam of cold light intended to pass through said second quartz crystal (45),
so as to obtain a treatment light beam which is governed by the passage through said second quartz crystal (45) and which is emitted at said apex (23),
which treatment device (1) comprises control means (5), to drive the operation of said light means (4),
which control means (5) include connection means (11) to said master treatment device (10).

2. The system for treating a subject according to claim 1, wherein the envelope (2) has a ratio of side dimension to height dimension ranging from 1.5 to 1.8.

3. The system for treating a subject according to claim 2, wherein the envelope (2) has:
- a side dimension ranging from 225 to 245 mm, and
- a height dimension ranging from 140 to 180 mm.

4. The system for treating a subject according to any one of claims 1 to 3, wherein the directional chamber (42) integrates an optical filter (47) that is arranged between the second quartz crystal (45) and the second cold light source (46).

5. The system for treating a subject according to any one of claims 1 to 4, wherein said treatment device (1) includes a plate (8) which is provided with first symbols and through which the second quartz crystal (45) passes,
which treatment device (1) includes means for mechanically connecting to a treatment table including second symbols, identical to said first symbols.

6. The system for treating a subject according to any one of claims 1 to 5, wherein said treatment device (1) includes means for electronic connection (12) to a human-machine interface, for example a portable electronic device, for driving said control means (5).

7. The system for treating a subject according to any one of claims 1 to 6, wherein the envelope (2) includes a removable apex part (2a), oriented coaxially to said central axis (2'), so as to obtain an opening in said envelope (2) at which the directional chamber (42) and said second quartz crystal (45) open and at which the treatment light beam is emitted.

8. The system for treating a subject according to claim 7, wherein the envelope (2) includes a recess (27) in its base (21), for housing said removable apex part (2a).

9. The system for treating a subject according to any one of claims 1 to 8, wherein the second quartz crystal (45) consists of a multifaceted quartz crystal (45).

10. The system for treating a subject according to claim 9 in combination with claim 5, also comprising said treatment table.
